(19) Europäisches Patentamt
European Patent Office
Office européen des brevets

(11) Publication number: 0 024 503
B1

(12) **EUROPEAN PATENT SPECIFICATION**

(45) Date of publication of patent specification: **15.06.83**

(51) Int. Cl.³: **C 07 C 85/00**, C 07 C 87/62

(21) Application number: **80103643.5**

(22) Date of filing: **27.06.80**

(54) **N-denitration of N,2,6-trinitroanilines with phase transfer catalysts.**

(30) Priority: **24.08.79 US 69451**

(43) Date of publication of application:
**11.03.81 Bulletin 81/10**

(45) Publication of the grant of the patent:
**15.06.83 Bulletin 83/24**

(84) Designated Contracting States:
**DE FR GB IT NL**

(56) References cited:
**DE - A - 2 429 958**
**GB - A - 2 008 092**
**US - A - 4 136 117**

(73) Proprietor: **AMERICAN CYANAMID COMPANY**
**1937 West Main Street P.O. Box 60**
**Stamford Connecticut 06904 (US)**

(72) Inventor: **Daniels, William Alan**
**114 Woods Road**
**Belle Mead New Jersey (US)**
Inventor: **Zawadzki, Rainer Kurt**
**Box 96A Provinceline Road R.D. 1**
**Hopewell New Jersey (US)**

(74) Representative: **Diehl, Hermann O., Dr. et al,**
**Diehl & Kressin Flüggenstrasse 17**
**D-8000 München 19 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 024 503 B1

N-denitration of N,2,6-trinitroanilines with phase transfer catalysts

The invention provides a procedure for the rapid elimination of a nitro group attached to the amino function of a secondary trinitroaniline of formula (I) in order to facilitate the removal of the nitro group from nitration mixtures obtained during the preparation of herbicidal dinitroaniline compounds of formula (II); both schematically illustrated below:

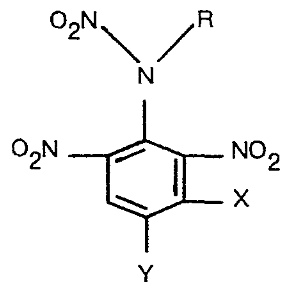

(I)

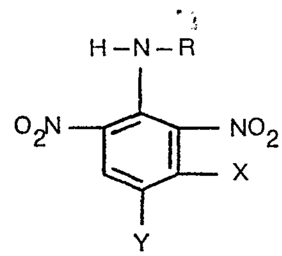

(II)

wherein R is $C_1$—$C_6$ alkyl (straight chain, or preferably branched, especially secondary alkyl), $C_4$—$C_6$ cycloalkyl, $C_1$—$C_4$ monohaloalkyl or $C_1$—$C_4$ alkoxy($C_2$—$C_4$)alkyl; Y is $C_1$—$C_4$ alkyl, halogen or $CF_3$; X is hydrogen, halogen, $C_1$—$C_4$ alkyl, $C_1$—$C_4$ alkoxy, $C_1$—$C_4$ monohaloalkyl or $C_1$—$C_4$ alkoxy($C_1$—$C_4$)alkyl; wherein the above denitration procedure comprises: reacting the formula (I) compound in the presence of a phase transfer catalyst with an aqueous solution of a strong base, as hereinbelow discussed and described in detail.

Among the compounds represented by formulae (I) and (II), a preferred group of compounds are those wherein R is $C_3$—$C_6$ alkyl (straight chain, or preferably a secondary alkyl); Y is $C_1$—$C_3$ alkyl or $CF_3$; X is hydrogen, $CH_3$ or $CH_3OCH_2$.

The most preferred compounds of formulae (I) and (II) are those wherein R is 1-ethylpropyl; Y is methyl or isopropyl; X is methyl or methoxymethyl.

The compounds of formula (I) are obtained as the unwanted byproducts of nitration reactions designed to prepare the herbicidal dinitroanilines of formula (II). It should be noted here that, in the course of the above-referred-to nitration reactions, N-nitroso-dinitroanilines of formula (IV) are formed too. The above-referred-to nitration reaction is hereinbelow schematically illustrated:

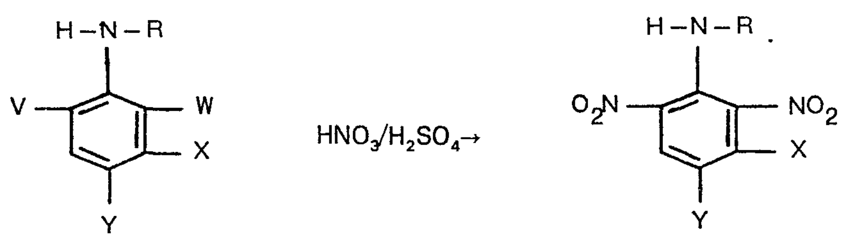


(III) (II)

The unwanted by-products of this reaction are:

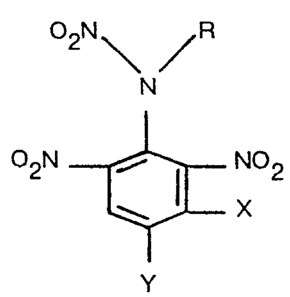

(I)

AND

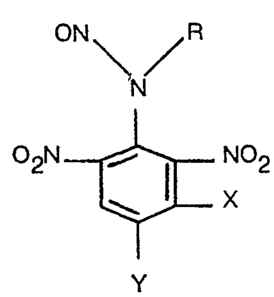

(IV)

wherein in the above reaction scheme, R, X and Y are as hereinabove defined; and V and W are both hydrogen, or if only one of them is hydrogen, then the other must be nitro.

The compounds of formula (III) may be nitrated conveniently by dissolving same in an inert water-immiscible solvent, such as ethylene dichloride, chloroform, carbon tetrachloride, monochlorobenzene, and the like, and preferably ethylene dichloride, and monochlorobenzene, and then the solution is added to a stirred nitration mixture consisting of nitric acid and sulfuric acid at a rate designed to control the ensuing exothermic reaction within the desired temperature limits. On completion of the reaction, the organic phase is separated. This phase contains in addition to the desired herbicides of formula (II) the N-nitro- (I) and N-nitroso- (IV) dinitroaniline impurities. Obviously, formation of compounds of formulae (I) and (IV) results in lower yields of the desired formula (II) herbicidal dinitroanilines. Advantageously, the formula (IV) impurities may be reconverted by chemical means to the desired formula (II) products, and, consequently, the yield of these herbicides (II) can be improved. More important is, however, the fact that these unwanted impurities, especially of formula (IV), may be either completely eliminated from, or at least lowered to acceptable levels in the products (II).

Thus, it is known that formula (IV) nitroso compounds may be denitrosated with sulfamic acid to the corresponding formula (II) pesticides either by treating the nitration mixture or the isolated organic phase with sulfamic acid. This denitrosation reaction may be carried out, however, more advantageously by the method of United States Patent No. 4,137,917, issued January 16, 1979 (Assignee: American Cyanamid Company), incorporated herein by way of reference, and illustrated in Example 5.

It should be noted here, that the thermal decomposition of formula (I) N-nitroanilines may also result in the formation of formula (IV) N-nitrosoanilines, as illustrated below:

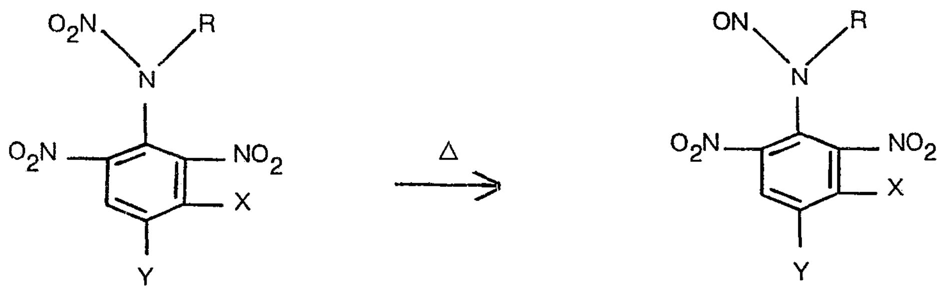

(I) (IV)

wherein R, X and Y are as hereinabove defined. The significance of this reaction is in that after the N-nitroso compounds of formula (IV) have been removed from the above-discussed nitration mixture or the product isolated therefrom, any formula (I) N-nitroaniline present in either the organic phase of the nitration mixture or in the product isolated therefrom, may thermally decompose and regenerate this undesirable contaminant (IV).

The N-denitration of secondary N-nitroamines is known to take place when these compounds are heated in aqueous potassium hydroxide. We find, however, that this denitration does not proceed at an acceptable rate with the N,2,6-trinitroanilines of formula (I) of the present invention.

Advantageously, by the novel method of the present invention, N-nitroanilines of formula (I) may be removed from the above-said nitration mixtures or the products isolated therefrom with bases in the presence of phase transfer catalysts.

Thus, the separated organic phase of a nitration mixture comprising: a solution of compounds of formulae (I), (II) and (IV) in a solvent, such as ethylene dichloride, chloroform, carbon tetrachloride, monochlorobenzene, and the like, preferably ethylene dichloride, is first denitrosated by the method of United States Patent No. 4,134,917 and is then mixed with a phase transfer catalyst selected from tetraalkyl phosphonium salts such as tetra-*n*-butyl phosphonium chloride or acetate, or from quaternary ammonium salts such as tetra-*n*-butylammonium salts, tricapryl methylammonium salts, benzyl trimethylammonium salts, benzyl triethylammonium salts, and the like, wherein said quaternary ammonium salts are the chlorides, bromides and iodides, and are used in amounts of from about 0.15 mole to about 2.5 moles, and preferably 1.0 mole to 1.2 moles per mole of said formula (I) compound, and then an aqueous base, selected from sodium or potassium hydroxide or carbonate, ammonium hydroxide or carbonate, is added in amounts sufficient to maintain a pH of 7 or more throughout the reaction. The resulting two-phase mixture is rapidly agitated at reflux by suitable means for a period of from 5 to 60 minutes, or until the reaction is essentially complete, and the amount of formula (I) compound originally present in said mixture is reduced to zero or to an acceptable level. Next, the two phases are separated, and the desired herbicidal 2,6-dinitroaniline of formula (II) is recovered from the organic phase.

Thus, for instance, 200 g of the organic phase of a nitration reaction, from which the N-(1-ethylpropyl)-2,6-dinitro-N-nitroso-3,4-xylidene has been removed by the denitrosation method of United States Patent No. 4,134,917; and now comprises: a solution of 120 g of N-(1-ethylpropyl)-2,6-dinitro-3,4-xylidine and 0.3 g of N-(1-ethylpropyl)-N,2,6-trinitro-3,4-xylidene in 80 g of ethylene

dichloride is mixed with 20 g of 10% sodium hydroxide solution and 0.36 g of benzyl triethylammonium chloride. The resulting two-phase reaction mixture is agitated rapidly by suitable means and heated at reflux for one hour, diluted with water, the organic phase separated, and the product isolated by standard laboratory procedures. The isolated product is found to be free of the corresponding formula (I) nitramine.

The invention of the present application is further demonstrated by the following non-limiting examples.

### Example 1

Denitration of N-(1-Ethylpropyl)-N,2,6-trinitro-3,4-xylidene

A solution of N-(1-ethylpropyl)-2,6-dinitro-3,4-xylidene (120 g) and N-(1-ethylpropyl)-N,2,6-trinitro-3,4-xylidene (0.3 g) in ethylene dichloride (80 g) is mixed with 10% sodium hydroxide solution (20 g) and benzyl triethylammonium chloride (0.36 g). The reaction mixture is stirred and heated at reflux for one hour. Water (100 g) is added, the phases are separated, and the aqueous phase washed with ethylene dichloride (2 x 50 ml). The organic phase and washings are combined, and the solvent evaporated under vacuum to afford 120.59 g solid. By analysis, this solid contains 97.6% by weight of N-(1-ethylpropyl)-2,6-dinitro-3,4-xylidine, 600 ppm of N-(1-ethylpropyl)-N,2,6-trinitro-3,4-xylidene and <60 ppm of N-(1-ethylpropyl)-2,6-dinitro-N-nitroso-3,4-xylidene.

### Example 2

Denitration of N-(1-Ethylpropyl)-N,2,6-trinitro-3,4-xylidine

To the isolated organic phase of a nitration mixture comprising: a solution of N-(1-ethylpropyl)-2,6-dinitro-3,4-xylidine (*a*), N-(1-ethylpropyl)-N,2,6-trinitro-3,4-xylidine (*b*), and N-(1-ethylpropyl-2,6-dinitro-N-nitroso-3,4-xylidine (*c*), in ethylene dichloride (weight of solution: 22.2 kg), is added a 15% aqueous sodium hydroxide solution (1.25 kg) and benzyl triethylammonium chloride (0.136 kg), and the mixture is stirred and heated at reflux for 2 hours. Next, water (11.32 kg) is added, the mixture stirred, and the two phases are then separated. A sample of the organic phase is taken and evaporated to dryness under vacuum, and the solid residue analyzed. The data obtained are compared to those obtained by analyzing the solids isolated from the above-referred-to organic phase of a nitration mixture, and are shown below:

Analysis

| Compound | Start | End |
|---|---|---|
| *a* | 94% | 93.3% |
| *b* | 7368 ppm | < 3 ppm |
| *c* | 130 ppm | 103 ppm |

It can be clearly seen from the above data, that the novel process of the present invention effectively removes the unwanted N-nitro compound (*b*) from the crude reaction mixture.

### Example 3

Denitration of N-(1-Ethylpropyl)-N,2,6-trinitro-3,4-xylidene

A solution of N-(1-ethylpropyl)-2,6-dinitro-3,4-xylidene (*a*), N-(1-ethylpropyl)-N,2,6-trinitro-3,4-xylidine (*b*), and N-(1-ethylpropyl)-2,6-dinitro-N-nitroso-3,4-xylidene (*c*) in ethylene dichloride (weight of solutions: 200 g), is mixed with a 10% aqueous solution of potassium hydroxide (20 g) and benzyl triethylammonium chloride (0.3164 g). The reaction mixture is stirred, heated to reflux in about 20 minutes and refluxed for one hour. Hot water (100 g at 82°C) is added, the mixture stirred for 5 minutes, and then the phases are separated. The thus-obtained organic phase is analyzed, and the data obtained are compared to those obtained by analyzing the solution prior to the denitration of same with the above base and phase transfer catalyst.

Analysis

| Compound | Start | End |
|---|---|---|
| *a* | 58.6% | not determined |
| *b* | 1065 ppm | <2 ppm |
| *c* | 9 ppm | 9 ppm |

Example 4

Denitration of N-(1-Ethylpropyl)-N,2,6-trinitro-3,4-xylidene

The experiment of Example 3 is repeated twice, using samples of the same solution, except that no benzyl triethylammonium chloride is added to the reaction mixtures. On completion of the reactions, the mixtures are worked up as in Example 3, and analyzed for N-(1-ethylpropyl)-2,6-dinitro-3,4-xylidene (*a*), N-(1-ethylpropyl)-N,2,6-trinitro-3,4-xylidene (*b*), and N-(1-ethylpropyl)-2,6-dinitro-N-nitroso-3,4-xylidine (*c*). The thus-obtained analytical data are compared to the values obtained by analyzing the starting material used in these runs (the starting material is the same as the one used in said Example 3).

Analysis

| Sample | (a) % | (b) ppm | (c) ppm |
|---|---|---|---|
| Starting Material | 58.6 | 1065 | 9 |
| Run I | not determined | 641 | 12 |
| Run II | 61.0 | 646 | 10 |

Example 5

Denitrosation of N-(1-Ethylpropyl)-2,6-dinitro-N-nitroso-3,4-xylidene with Diethyl Ketone

A glass pressure reactor is charged with an ethylene dichloride solution (96.0 g) of N-(1-ethylpropyl)-2,6-dinitro-3,4-xylidene (38.2% by weight = 36.7 g) and N-(1-ethylpropyl)-2,6-dinitro-N-nitroso-3,4-xylidene (13.4% by weight = 12.9 g), concentrated hydrochloric acid (14.7 g) and diethyl ketone (2.5 g), and the reactor is then sealed. The reaction mixture is heated to 80—85°C and stirred for 4 hours while the temperature is maintained at 80—88°C. The pressure increases within the vessel to a maximum of 1.68 kg/cm$^2$ and then slowly decreases to 1.26 kg/cm$^2$ in the course of the reaction. On completion of the reaction, the reactor is cooled down, vented, the pH of the reaction mixture adjusted to 10 with 10% sodium hydroxide and filtered. The filtercake is washed several times with ethylene dichloride, the organic layer separated, and the solvent removed under vacuum to afford 51.4 g of solid containing (by analysis) 93.3% by weight of N-(1-ethylpropyl)-2,6-dinitro-3,4-xylidene (47.9 g) and <0.01% by weight of N-(1-ethylpropyl)-2,6-dinitro-N-nitroso-3,4-xylidene (0.51 g).

Example 6

Preparation of N-(1-Ethylpropyl)-2,6-dinitro-3,4-xylidine

A nitration mixture is prepared by adding 70.5% nitric acid (145.2 g; 1.625 mol) and 94.5% sulfuric acid (116.2 g; 1.12 mol) to water (58.8 g). To the above-mixed acid, a solution of 94.6% pure N-(1-ethylpropyl)-3,4-xylidene (101.0 g; 0.5 mol) in ethylene dichloride (143.5 ml) is added over a period of 2 hours at 35°C. The reaction mixture is stirred at 35°C for one hour, and the aqueous phase is then separated. The organic phase is washed successively with 5% sodium hydroxide (300 ml) and water (300 ml) and is then dried over magnesium sulfate. The organic solution is then concentrated under vacuum at 70°C to yield 141.5 g of a solid containing 117.0 g (72.6%) of title product.

By the above procedure, but substituting monochlorobenzene for ethylene dichloride, the title product is obtained in similar yields and purity.

Example 7

Denitration of N-(1-Ethylpropyl)-N,2,6-trinitro-3,4-xylidine

The separated organic phase of a nitration mixture obtained by the procedure of Example 6, wherein the solvent of said organic phase is monochlorobenzene, is first denitrosated by the procedure of Example 5, and is then denitrated by the procedure of Example 2. The organic phase is analyzed before and after the denitration reaction. The data obtained are shown below:

Analysis

| Compound | Start | End |
|---|---|---|
| *b* | 1000 ppm | 65 ppm*<br><2 ppm** |
| *c* | <20 ppm | 6 ppm*<br>7 ppm** |

*In 30 minutes at 70°C.
**After an additional 30 minutes at 87°C.

A second experiment, performed as above, yields the following data:

Analysis

| Compound | Start | End |
|---|---|---|
| *b* | 2750 ppm | <2 ppm*<br><2 ppm** |
| *c* | 42 ppm | 14 ppm*<br>14 ppm** |

*In 30 minutes at 70°C.
**After an additional 30 minutes at 87°C.

In the above Examples, *b* and *c* have the same definition as in Example 2.

**Claims**

1. A process for the N-denitration of a compound of formula:

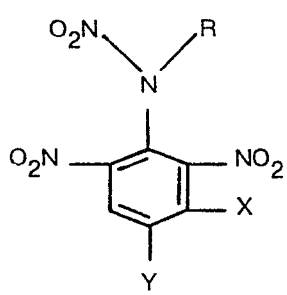

wherein R is $C_1$—$C_6$ alkyl, $C_4$—$C_6$ cycloalkyl, $C_1$—$C_4$ monohaloalkyl or $C_1$—$C_4$ alkoxy($C_2$—$C_4$)alkyl; Y is $C_1$—$C_4$ alkyl, halogen or $CF_3$; X is hydrogen, halogen, $C_1$—$C_4$ alkyl, $C_1$—$C_4$ alkoxy, $C_1$—$C_4$ monohaloalkyl or $C_1$—$C_4$ alkoxy($C_1$—$C_4$)alkyl; comprising: reacting the compound with an aqueous solution of an alkali metal hydroxide or carbonate or ammonium hydroxide in amounts sufficient to maintain at least pH 7 throughout the reaction in the presence of 0.15 to 2.5 molar amount of a phase transfer

catalyst selected from tetralkyl phosphonium salts such as tetra-*n*-butyl phosphonium chloride or acetate or tetra-*n*-butylammonium salt, tricapryl methylammonium salt, benzyl trimethylammonium salt or benzyl triethylammonium salt; in the presence of a water-immiscible solvent of ethylene dichloride, chloroform, monochlorobenzene or carbon tetrachloride; and wherein the thus-obtained two-phase reaction mixture is agitated at reflux until the reaction is essentially complete.

2. A process according to Claim 1 wherein R is $C_3$—$C_6$ alkyl, Y is $C_1$—$C_3$ alkyl or $CF_3$, X is hydrogen, $CH_3$ or $CH_3OCH_2$—.

3. A process according to Claim 2 wherein the compound is N-(1-ethylpropyl)-N,2,6-trinitro-3,4-xylidine.

4. A process according to Claim 3 wherein the phase transfer catalyst is benzyl triethylammonium chloride, the solvent is ethylene dichloride, and the base is sodium hydroxide.

5. A process according to Claim 4 wherein the phase transfer catalyst used in 1.0 to 1.2 molar amounts.

6. A process according to Claim 2 wherein the solvent is monochlorobenzene.

**Patentansprüche**

1. Verfahren zur N-Denitrierung der Verbindung der allgemeinen Formel

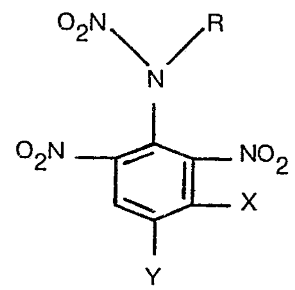

worin R für $C_1$—$C_6$-Alkyl, $C_4$—$C_6$-Cycloalkyl, $C_1$—$C_4$-Monohalogenalkyl oder $C_1$—$C_4$-Alkoxy($C_2$—$C_4$)alkyl steht, Y für $C_1$—$C_4$-Alkyl, ein Halogenatom oder $CF_3$ steht, X für ein Wasserstoffatom, Halogenatom, $C_1$—$C_4$-Alkyl, $C_1$—$C_4$-Alkoxy, $C_1$—$C_4$-Monohalogenalkyl oder $C_1$—$C_4$-Alkoxy($C_1$—$C_4$)alkyl steht, dadurch gekennzeichnet, daß man die Verbindung der obigen allgemeinen Formel mit einer wäßrigen Lösung eines Alkalimetallhydroxids oder -carbonats oder Ammoniumhydroxid in einer so ausreichenden Menge umsetzt, daß der pH-Wert während der Umsetzung wenigstens bei 7 eingestellt ist, und daß man die Umsetzung mit einer 0,15 bis 2,5 molaren menge eines Phasenumkehrkatalysators, ausgewählt aus der Gruppe bestehend aus Tetraalkylphosphoniumsalzen, wie Tetra-n-butylphosphoniumchlorid oder -acetat oder einem Tetra-n-butylammoniumsalz, Tricarprylmethylammoniumsalz, einem Benzyltrimethylammoniumsalz oder einem benzyltriethylammoniumsalz, in Gegenwart von Ethylendichloride, Chloroform, Monochlorbenzol oder Tetrachlorkohlenstoff als wasserunmischbares Lösungsmittel vornimmt, und daß man die so hergestellte Zweiphasen-Reaktionsmischung unter Rückfluß rührt, bis die Umsetzung im wesentlichen beendet ist.

2. Verfahren nach Anspruch 1, dadurch gekennzeichnet, daß R für $C_3$—$C_6$-Alkyl, Y für $C_1$—$C_3$-Alkyl oder $CF_3$, und X für ein Wasserstoffatom, $CH_3$ oder $CH_3OCH_2$— steht.

3. Verfahren nach Anspruch 2, dadurch gekennzeichnet, daß die Verbindung der allgemeinen Formel N-(1-ethylpropyl)-N,2,6-trinitro-3,4-xylidin ist.

4. Verfahren nach Anspruch 3, dadurch gekennzeichnet, daß man als Phasenumkehrkatalysator Benzyltriethylammoniumchlorid, als Lösungsmittel Ethylendichlorid und als Base Natriumhydroxid verwendet.

5. Verfahren nach Anspruch 4, dadurch gekennzeichnet, daß man den Phasenumkehrkatalysator in einer 1,0 bis 1,2 molaren Menge verwendet.

6. Verfahren nach Anspruch 2, dadurch gekennzeichnet, daß man als Lösungsmittel Monochlorbenzol verwendet.

**Revendications**

1. Un procédé de N-dénitration d'un composé de formule:

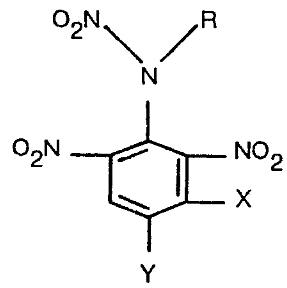

dans laquelle R est un groupe alkyle en $C_1$—$C_6$ cycloalkyle en $C_4$—$C_6$, monohaloalkyle en $C_1$—$C_4$ ou alcoxy ($C_1$—$C_4$)-alkyle ($C_2$—$C_4$); Y est un groupe alkyle en $C_1$—$C_4$, un halogène ou $CF_3$; X est l'hydrogène, un halogène ou un groupe alkyle en $C_1$—$C_4$, alcoxy en $C_1$—$C_4$, monohaloalkyle en $C_1$—$C_4$ ou alcoxy ($C_1$—$C_4$-alkyle ($C_1$—$C_4$); comprenant la réaction du composé avec une solution aqueuse d'un hydroxyde ou carbonate de métal alcalin ou d'hydroxyde d'ammonium en quantités suffisantes pour maintenir le pH à au moins 7 tout au long de la réaction en présence d'une quantité de 0,15 à 2,5 molaire d'un catalyseur de transfert de phase choisi parmi des sels de tétralkyl phosphonium tels que le chlorure ou l'acétate de tétra-n-butylphosphonium ou un sel de tétra-n-butylammonium, un sel de tricapryl méthylammonium, un sel de benzyl triméthylammonium ou un sel de benzyl triéthylammonium; en présence d'un solvant non miscible à l'eau qui est le dichlorure d'éthylène, le chloroforme, le monochlorobenzène ou le tétrachlorure de carbone; et dans lequel le mélange réactionnel à deux phases ainsi obtenu est agité au reflux jusqu'à ce que la réaction soit pratiquement achevée.

2. Un procédé selon la revendication 1 dans laquel R est un groupe alkyle en $C_3$—$C_6$, Y est un groupe alkyle en $C_1$—$C_3$ ou $CF_3$, X est l'hydrogène, $CH_3$ ou $CH_3OCH_2$.

3. Un procédé selon la revendication 2 dans laquel le composé est la N-(1-éthylpropyl)-N,2,6-trinitro-3,4-xylidine.

4. Un procédé selon la revendication 3 dans lequel le catalyseur de transfert de phase est le chlorure de benzyl triéthylammonium, le solvant est le dichlorure d'éthylène, et la base est l'hydroxyde de sodium.

5. Un procédé selon la revendication 4 dans laquel le catalyseur de transfert de phase est utilisé en quantités molaires de 1,0 à 1,2.

6. Un procédé selon la revendication 2 dans laquel le solvant est le monochlorobenzène.